# FASCICULE DE BREVET EUROPEEN

(11) **EP 0 765 192 B1**
(45) Date de publication et mention de la délivrance du brevet: **29.03.2000**
(21) Numéro de dépôt: 96901829.0
(22) Date de dépôt: 22.01.1996
(51) Int. Cl.: B01J 23/62, C22C 13/00, C07C 45/41

(54) **CATALYSEUR INTERMETALLIQUE RUTHENIUM-ETAIN UTILE DANS LA SYNTHESE D'ALDEHYDES**
INTERMETALLISCHER RUTHENIUM-ZINN-KATALYSATOR ZUR SYNTHESE VON ALDEHYDEN
INTERMETALLIC RUTHENIUM-TIN CATALYST FOR USE IN ALDEHYDE SYNTHESIS

(30) Priorité: 23.01.1995 FR 9500699
(43) Date de publication de la demande: 02.04.1997
(73) Titulaire: RHODIA CHIMIE, 92408 Courbevoie Cédex (FR)
(72) Inventeur: JACQUOT, Roland, F-69110 Sainte-Foy-les-Lyon (FR); LECLERCQ, Jean-Marc, F-95330 Domont (FR); MERCIER, Claude, F-69005 Lyon (FR); POPA, Jean-Michel, F-93700 Drancy (FR)
(74) Mandataire: Ricalens, François
(86) Numéro de dépôt international: FR9600101
(87) Numéro de publication internationale: WO9622832

(56) Documents cités:
- EP-A- 0 539 274
- EP-A- 0 626 201
- CHEMICAL ABSTRACTS, vol. 86, no. 26, 27 Juin 1977 Columbus, Ohio, US; abstract no. 198283m, HEINRICH: "Bonding in iron-tin and ruthenium-tin mixtures" page 565; XP002003086 & J. LESS-COMMON METALS, vol. 52, no. 1, GER, pages 87-91,
- JOURNAL OF CATALYSIS, vol. 121, no. 1, DULUTH, MN, US, pages 174-182, XP002003085 V.M. DESPHANDE ET.AL.: "Studies on ruthenium-tin boride catalysts" cité dans la demande

## Description

La présente invention concerne un procédé pour préparer des aldéhydes et leurs dérivés, par réduction en phase vapeur en présence d'hydrogène, d'acides, d'esters ou d'anhydrides carboxyiques.

Plus particulièrement, elle concerne un procédé de synthèse d'aldéhydes par réduction en phase vapeur d'acides, d'esters ou de leurs dérivés en présence d'un catalyseur bimétallique de type ruthénium/étain. Elle vise aussi un catalyseur et son utilisation pour la réduction sélective de dérivés carboxyliques en aldéhydes.

Le présent procédé vise plus particulièrement comme substrat les composés carboxyliques porteur d'halogène et notamment de fluor susceptibles d'être hydrogénolysés au cours de la réduction.

Il est connu dans l'art antérieur de préparer des aldéhydes aromatiques ou aliphatiques saturés par réduction des acides ou esters correspondants au moyen d'un catalyseur choisi parmi les oxydes de cérium, zirconium, uranium, praséodyme et yttrium, à une température comprise entre 350 et 450°C (US-A-4,328,373).

Compte tenu des conditions de températures requises pour leur mise en oeuvre, ces procédés ne permettent pas de préparer des aldéhydes à partir d'acides thermolabiles.

La demande de brevet publiée sous le numéro 2 682 949 décrit une technique utilisant des alliages de ruthénium et d'étain permettant un progrès significatif de la réduction des acides en aldéhydes. Cette demande ne décrit toutefois, ni l'existence, ni les conditions permettant l'obtention d'une phase catalytique optimale, à savoir, une phase comportant le composé intermétallique Ru₃/Sn₇.

Des catalyseurs de type Ru/Sn/B avait déjà été décrits dans la littérature [J. Cat. 121, 165-173 (1990)], ainsi que leur utilisation pour la réduction, en phase liquide, d'acides gras insaturés en alcools gras insaturés [J. Cat., 121, 174-182 (1990)].

Certains problèmes restaient non résolus. Pour un certain nombre de substrats donnant lieu à des réactions parasites le choix du support et de la manière dont était constituées la couche catalytique superficielle s'est révélée essentielle. Le problème est particulièrement aigu lors de la réduction de certains dérivés halogénés dont la lyse par l'hydrogène conduit à des acides particulièrement agressifs tels que par exemple l'acide fluorhydrique.

Ces acides sont capables de détruire certains supports et perturbent la réaction désirée. Un autre risque réside dans une catalyse de la réduction de l'aldéhyde en alcool. C'est pourquoi un des buts de la présente invention est de fournir une composition utile comme catalyseur qui permette une résistance améliorée aux produits de réaction(s) parasite(s).

Un autre but de la présente invention est de fournir un composition du type précédent qui évite les réactions parasites ou en réduise l'importance relative.

Ces buts et d'autres qui apparaîtront par la suite sont atteints au moyen d'une composition utile comme catalyseur de réduction, laquelle comporte un support qui comporte comme matériau constitutif au moins un oxyde choisi parmi les oxydes inertes ou susceptibles d'être rendu inertes vis à vis du mélange réactionnel et une phase métallique recouvrant au moins en partie ledit support comportant au moins en partie un intermétallique ruthénium-étain au moins partiellement sous forme du composé défini de Ru₃Sn₇, le rapport Sn/Ru de la dite phase métallique étant au moins égal à 3/2 et au plus égal à 3, et par le fait que ladite phase contient au moins 50 % de phase Ru₃Sn₇.

Avantageusement la phase contenant le ruthénium et l'étain présente un rapport atomique Sn/Ru au moins égal à 2/3, avantageusement à 3/2, de préférence à 7/3. Par ailleurs il est préférable que le rapport atomique Sn/Ru soit au plus égal à 3, avantageusement à 5/2.

Avantageusement ladite phase recouvrant au moins en partie ledit support contient au moins 50 % , avantageusement 80%, de préférence au moins 90% de ladite phase intermétallique.

Enfin il est souhaitable qu'au moins 90 %, avantageusement au moins 95%, de préférence 98 % du ruthénium présent sur le support soit sous la forme de ladite phase recouvrant ledit support.

L'invention est plus particulièrement adaptée à la préparation d'aldéhydes de formule générale: 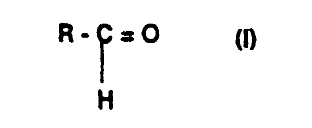 dans laquelle R représente un atome d'hydrogène ou un radical hydrocarboné, éventuellement substitué comportant de 1 à 40 atomes de carbone qui peut être un radial aliphatique acyclique saturé ou insaturé, linéaire ou ramifié ; un radical carbocyclique ou hétérocyclique saturé, insaturé ou aromatique, monocyclique ou polycyclique, par réduction d'esters, d'anhydrides ou d'acides de formule: 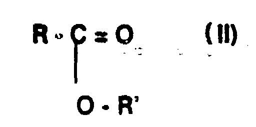 dans laquelle :
- R est défini comme précédemment,
- R' représente:
   - un groupement R tel que précédemment défini,
   - un groupement 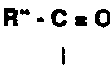 dans lequel R" a la signification donnée pour R,
   - les deux groupements R et R" pouvant être liés entre eux pour former un cycle saturé ou insaturé ayant de 5 à 7 atomes et comprenant la fonction anhydride,
   - les deux groupements R et R" par l'intermédiaire de deux atomes vicinaux pouvant former ensemble un pont d'un système bicyclique ortho-condensé.

Les acides carboxyliques ou dérivés mis en oeuvre préférentiellement répondent à la formule (Il) dans laquelle R représente un radical hydrocarboné, éventuellement substitué comportant de 1 à 20 atomes de carbone.

L'invention convient tout à fait bien, à la préparation d'aldéhydes à partir d'acides carboxyliques aliphatiques halogénés tels que le fluoral.

L'invention est très bien adaptée à la synthèse d'aldéhydes à partir d'acides carboxyliques aromatiques et des acides halogénobenzoïques, de préférence des acides fluorobenzoïques.

Dans l'exposé qui suit de la présente invention, on entend par composé aromatique, la notion classique d'aromaticité telle que définie dans la littérature, notamment par Jerry MARCH - Advanced Organic Chemistry, 3ème édition, John Wiley and Sons. 1985 p. 37 et suivantes.

On entend par acide benzoïque, tout composé benzénique portant au moins une fonction COOH.

Comme indiqué précédemment, il est également possible de mettre en oeuvre l'acide carboxylique tel que défini ci-dessus sous la forme de son ester. Dans ce cas, dans la formule (II), R' représente de préférence un radical aliphatique contenant de 1 à 10 atomes de carbone, éventuellement substitué. Plus préférentiellement, R' représente un radical alcoyle linéaire ou ramifié ayant de 1 à 6 atomes de carbone.

A titre d'exemples de radicaux R' préférés, on peut citer les radicaux méthyle, éthyle ou hexyle.

L'invention est particulièrement bien adaptée à la synthèse d'aldéhydes présentant dans leur formules un ou plusieurs halogènes, surtout quand ces halogènes sont au moins en partie des fluors.

Sont particulièrement visés les aldéhydes obtenus à partir des acides perhalogénocarboxyliques ou ceux qui leurs sont équivalents sur le plan de la réactivité. Sont donc équivalents des perhalogénocarbaxyliques les acides dont le carbone vicinal, ou plutôt les deux carbones vicinaux de la fontion carboxylique sont perhalogénés. Ainsi, l'invention est particulièrement bien adaptée à la synthèse d'aldéhydes où le, ou plutôt les deux carbones, vicinaux sont perfluorés.

Conformément à la présente invention, on peut faire appel à un acide carboxylique sous la forme de ses anhydrides et esters.

Comme exemples d'anhydrides carboxyliques, on peut mentionner plus particulièrement les homos-anhydrides qu'ils soient internes (les anhydrides cycliques) ou non et les hétéroanhydrides (ou anhydrides mixtes).

Les composés préférés sont bicycliques et constitués d'un cycle benzénique

Le procédé de l'invention est mis en oeuvre en phase gazeuse. Avantageusement, la réaction est conduite à une température comprise erre 100°C et 500°C, et encore plus préférentiellement entre 200 et 400°C. Il est entendu que la température est adaptée par l'homme de l'art en fonction de l'acide de départ, et de la vitesse de réaction recherchée.

Comme cela a été mentionné ci-dessus, il est très souhaitable que la phase contenant le ruthénium et rétain présente un rapport atomique Sn/Ru au moins égal à 3/2, de préférence égal à 7/3. Par ailleurs le rapport atomique Sn/Ru est au plus égal à 3, avantageusement à 5/2.

Au cours de l'étude qui a mené à la présente invention, pour obtenir de telles valeurs, il a été montré combien il était important de réaliser une réduction poussée à relativement haute température de manière à atteindre les proportions ci-dessus en étain. En effet dans les proportions ci-dessus les teneurs en étain ne tiennent compte que de l'étain métallique.

Dans les documents de l'état antérieur de la technique, il n'a été décrit que des techniques douces de réduction qui semblent être très insuffisantes pour réduire l'étain de manière à se rapprocher de la zone optimum correspondant au composé intermétallique Ru₃Sn₇.

En effet, il a été montré que, pour obtenir des effets optimaux, la réduction décrite dans les documents antérieurs était insuffisante, en sorte que l'intermédiaire Ru₃Sn₇ s'il existait, était dans des proportions trop faibles pour jouer son rôle de catalyseur sélectif.

Il est possible d'obtenir une phase intermétallique selon la présente invention (ou d'un gaz source d'hydrogène dans les conditions de procédé tel que le propylène et comme l'éthylène) par imprégnation du support par des sels ou des oxydes, que l'on soumet à une réduction par un courant d'hydrogène (par exemple sous 2. 10⁵ Pa au moins). à une température d'au moins 400°C de préférence d'au moins 450° C. L'imprégnant présente un proportion d'étain au moins égale à celle de Ru₃Sn₇, de préférence significativement supérieure.

Le traitement doit être d'autant plus long que les températures sont moins élevées. A une température de 400°C un jour est un minimum, alors que ce minimum n'est que de 5 heures environ à 450°C. Dans un premier temps il semble que le ruthénium catalyse la réduction de l'étain et que, dans ces conditions, rétain excédentaire soit au moins partiellement éliminé et que la phase intermétallique sur le support se rapproche de la composition de Ru₃Sn₇. Plus cette étape dure, plus on se rapproche de la composition optimale du catalyseur, à condition d'être parti d'un excès d'étain.

Une manière pratique de mettre en oeuvre la présente invention consiste à introduire dans un réacteur une quantité désirée de catalyseur. La température du réacteur est ensuite élevée sous courant d'hydrogène jusqu'à une valeur déterminée, permettant d'activer le catalyseur, puis ramenée à la température de réaction. L'acide est ensuite injecté au débit souhaité et l'aldéhyde formé est récupéré.

Préférentiellement, l'acide est injecté directement sous forme gazeuse après avoir été vaporisé par chauffage.

Toutefois, il peut également être injecté en solution dans un solvant inerte pour la réaction. On peut citer en particulier comme solvants inertes les hydrocarbures aliphatiques (par exemple l'hexane), alicycliques (par exemple le cyclohexane). aromatiques (par exemple le toluène), ou les éthers (par exemple la diméthoxyéthane). Sous l'effet de la température, l'acide ainsi injecté est vaporisé. L'hydrogène peut être injecté à la pression atmosphérique ou sous une légère pression compatible avec la phase vapeur (pression partielle de quelques bars, par exemple de 0,5 à 10 bar, le bar est ici réputé représenter 10⁵ Pa). L'hydrogène peut également être dilué dans un gaz inerte dans les conditions opératoires, tel que l'azote ou l'hélium.

Avantageusement, pour 1 ml de catalyseur, l'hydrogène est injecté à un débit compris entre 0,1 et 10 filtres par heure, et l'acide à un débit liquide au plus égal à 10 ml/h, et de préférence compris entre 0,5 et 5 ml/h.

En fin de réaction, on récupère l'aldéhyde par tout moyen approprié tel que distillation ou cristallisation. Dans certains cas, notamment dans le cas du fluoral, l'aldéhyde peut être obtenu sous une forme hydratée.

La teneur en élément(s) étranger(s) tel le bore est généralement inférieure à 1 % et de préférence inférieure à 0,1 % molaire.

Généralement, on dissout dans reau les deux métaux sous forme de sels, éventuellement en présence du support, et on laisse l'imprégnation s'effectuer sur une période de 15 heures environ. Celui-ci est ensuite séché (par exemple sous vide) avant son emploi.

L'un de ses procédés de préparation consiste, par exemple, à introduire un support dans une solution que l'on prépare en dissolvant au moins un composé approprié des éléments choisis ; le dépôt des éléments actifs sur le support est réalisé en distillant le solvant de préférence reau qui peut être éliminée par évaporation sous pression réduite choisie de préférence entre 5 et 20 mn de mercure. La masse de contact ainsi obtenue est soumise à une réduction au moyen d'un courant d'hydrogène.

Selon un autre mode de préparation classique, le dépôt du ou des composés apportant les éléments métalliques sur le support sont réalisés au moyen de composés préalablement précipités de manière en soi connue et en soumettant la masse de contact ainsi obtenue à une réduction au moyen de l'hydrogène.

Le dépôt sur le support de plusieurs éléments métaliques peut bien entendu être réalisé successivement mais de préférence simultanément.

Le dépôt des précurseurs de la phase intermétallique peut être fait par réitération d'un des processus décrit ci-dessus. Cette reitération peut être menée soit pour imprégnations itératives ou pour des cycles d'imprégnation-réduction itératifs.

La nature des composés apportant les éléments métalliques utilisés pour la préparation des catalyseurs de l'invention n'est pas critique à condition qu'il n'y ait pas de risque de modification du rapport étain/ruthénium avant que la grande majorité (au moins 3/4, avantageusement 9/10, de préférence 95 %) du ruthénium ne soit engagé dans une phase métallique avec l'étain.

On peut faire appel aux métaux eux-mêmes tels que le ruthénium et rétain.

A titre d'exemples de composés susceptibles d'être mis en oeuvre pour la préparation des catalyseurs de l'invention, on peut citer, à titre de composés du ruthénium, le chlorure de ruthénium III, le chlorure de ruthénium IV, le pentafluorure de ruthénium, l'oxyde de ruthénium II, l'oxyde de ruthénium IV, l'oxychlorure de ruthénium ammoniaqué Ru₂(OH)₂Cl₄, 7NH₃, 5H₂O, l'acétate de ruthénium et, à titre de composés de l'étain, les oxydes, chlorures, nitrates, carboxylates, alcoolates d'étain ou des composés organométalliques dans lesquels l'étain est lié à un atome d'hydrogène et/ou des radicaux alcoyle ayant de préférence de 1 à 4 atomes de carbone. Les sels préférés sont les suivants : les composés du ruthénium tels que le chlorure de ruthénium III, les composés de l'étain tels que la chlorure d'étain II, le chlorure d'étain IV, l'acétate d'étain II, l'octoate d'étain Il, l'éthylhexanoate d'étain.

On ne sortira pas du cadre de la présente invention à fabriquer selon le procédé de l'invention, les aldéhydes sous la forme de leurs dérivés, tels que leurs acétals, leurs hémiacétals ou leurs combinaisons bisulfitiques, par réaction de l'aldéhyde et du réactif [alcool dans le cas des (hémi)acétals] qui est introduit soit conjointement à l'acide, lorsque le réactif est volatil; soit en fin de réaction. Comme exemples d'alcools classiquement utilisés, on peut citer le méthanol ou l'éthanol.

Un des aspects les plus importants de l'invention réside dans le choix du support.

Il est souhaitable que ledit support présente une granulométrie telle que son d₂₀ soit au moins égal à 1 micromètre, avantageusement à 10 micromètres, de préférence à 50 micromètres.

Il est préconisé que la surface spécifique du dit support soit au plus égale à 10 fois de préférence à 5 fois la surface dite géométrique. Lorsque la surface spécifique est au moins égale à deux fois la surface dite géométrique, à savoir celle calculée géométriquement en faisant l'hypothèse que toutes les particules sont sphériques, au moins 20 %, avantageusement au moins 30 %, de préférence au moins 50 %, de l'excès de surface est attribuable à des pores de diamètre moyen au moins égal à 0,5 micromètre, avantageusement au moins égal à 1 micromètre, de préférence au moins égal à 5 micromètres.

Le support doit être choisi de manière à maximiser la résistance aux conditions industrielles, et en particulier la résistance aux abrasions mécanique ou quasi mécaniques; en particulier la résistance à l'attrition.

Le support doit être choisi de manière à éviter des pertes de charge importantes, tout en permettant un bon contact entre les gaz et le catalyseur.

Le support doit être choisi parmi les oxydes inertes (c'est à dire présentant une bonne résistance chimique vis à vis) ou susceptible d'être rendu inertes vis à vis du mélange réactionnel. Le problème est particulièrement aigu dans le cas où l'on choisit comme substrat des acides carboxyliques présentant des atomes d'halogènes et notamment de fluor.

Dans ce cas il est préférable que le support manifeste une résistance significative aux acides halohydriques humides et en particulier vis à vis de l'acide fluorhydrique gazeux humide (la réduction en aldéhyde d'un acide donne de l'eau).

Ledit support est avantageusement un support susceptible d'être rendu inerte par l'action de l'acide fluorhydrique gazeux sans perdre sa géométrie exteme et sa résistance mécanique.

Les diverses silices ne résistent pas à l'action de l'acide fluorhydrique.

Il est à noter que les supports de type charbon sont particulièrement à éviter car ils favorisent les réactions parasites et donnent lieu à une cokéfaction intense des substrats.

Comme oxydes de métaux, on peut choisir des oxydes tels que les oxydes d'aluminium, ou de zirconium. Les oxydes mixtes conviennent également ,plus particulièrement ceux contenant au moins 1/4 avantageusement 1/3, de préférence 2/5 en masse d'aluminium exprimé en Al₂O₃.

Ledit support présente avantageusement une teneur en silicium qui exprimée en SiO2 soit au plus égale à 2/3 du poids total. Avantageusement au plus égale 1/4.

Les céramiques obtenues par cuisson, à environ 1000°C au moins. des argiles contenant comme éléments principaux les oxydes d'aluminium et de silicium ont été testées et ont donné des résultats particulièrement intéressants. Comme argile silico-alumineuse donnant de bons résultats on peut citer les argiles de Provins, notamment celles vendues par Denain Anzin Minéraux sous la dénomination commerciale de chamotte 40/42.

Ledit support présente avantageusement une granulométrie telle que son d₂₀ soit au moins égal à 0,1 millimètre, avantageusement à 0,5 millimètre, de préférence à un millimètre.

Un bon arbitrage consiste à choisir des billes peu poreuses de 1mm à 1cm donnant de bon résultats.

Une surface spécifique du support avantageusement faible, inférieure à 10 m² par gramme, de préférence au plus égale à 1 m² par gramme.

Le rapport massique entre le support et la phase superficielle est le plus souvent compris entre les valeurs 0,5 % et 30 %, est avantageusement de 1 % à 15 %, de préférence de 2 % à 10 % ; ainsi des valeurs comprises entre 0,5 % et 10 %, plus avantageusement allant de 0,5 % à 5 %, de préférence de 1 % à 5 %, donnent de bons résultats.

Un autre but de la présente invention est de fournir un procédé qui permette de préparer la composition selon la présente invention.

Un autre but de la présente invention est de fournir un procédé du type précédent qui permette d'atteindre une phase de couverture aussi proche que possible de l'intermétallique ruthénium-étain de composition Ru3Sn7.

Ces buts et d'autres qui apparaîtront par la suite sont atteints au moyen d'un procédé de production de catalyseur qui comporte un support et une phase métallique recouvrant au moins en partie ledit support, le rapport Sn/Ru de ladite phase métallique étant au moins égal à 3/2 et au plus égal à 3, ladite phase contient au moins 50 % de phase Ru₃/Sn₇.
a) enrobage du support au moyen d'une solution ou d'une suspension d'espèces stannifère(s) et ruthénifère(s), le rapport atomique Sn/Ru étant au moins égal à 2,3 avantageusement à 3, de préférence à 4 ;
b) traitement à une température au moins égale à 400°, avantageusement à 450°C, sous une pression partielle d'hydrogène au moins égale à 1/2 10⁵ Pa, avantageusement comprise entre 1 et 10 bar, de préférence entre un et deux bars et ce pendant une durée d'au moins environ cinq heures, de préférence au moins 10 heures.

Il est souhaitable que le choix des espèces stannifère(s) et ruthénifère(s) soient tels que cela minimise la volatilisation pendant l'étape de réduction.

Les exemples non limitatifs suivants illustrent l'invention:

### CATALYSEUR I:

### Exemple 1 : Fabrication d'un lot de 9 litres de catalyseur 3,5 % Ru/Bille ex-argiles avec un rapport Sn/Ru de 2,3

### Préparation de la suspension d'hydroxyde d'étain

L'hydroxyde d'étain est obtenu par neutralisation à l'ammoniaque de SnCl₄, 5H₂O. On dissout 5 kg de SnCl₄, 5H₂O dans 3,1 litres d'eau.

On prépare ensuite 13.45 litres de solution 5 M d'ammoniaque par dilution de 5 litres d'ammoniaque à 28 %.

Juste avant neutralisation à l'ammoniaque de la solution de sel d'étain, on dilue cette solution avec 8,6 litres d'eau pour atteindre une concentration en sel d'étain de 1,25 M. La précipitation en régime permanent et en continu est effectuée dans un réacteur d'un litre équipé d'une agitation. d'une régulation de température, d'une régulation de pH en continu, d'une régulation de niveau et de deux pompes d'alimentation.

En aval de ce réacteur de précipitation est situé un réacteur "tampon" de 10 litres pour le stockage.

On part d'un pied de cuve initial d'eau épurée de 1 litre.

L'alimentation de la solution de chlorure d'étain s'effectue à débit constant de 2,5 l/h. L'alimentation de la solution d'ammoniaque dépend de la consigne de pH imposé et de l'écart à cette consigne. Le pH de précipitation choisi est de 5. L'agitation est réglée à 700 tr/min et la température est régulée à 30°C.

Une fois atteint la consigne deniveau, la suspension est aspirée hors du réacteur. Ce n'est qu'après 5 temps de passage que le réacteur est considéré en régime permanent et que le gel obtenu est recueilli dans le réacteur tampon.

La filtration est effectuée sur Buchner pour évacuer les eaux-mères. Le gel est ensuite repulpé dans 20 litres d'eau épurée et agité pendant 6 heures. La suspension est décantée pendant 12 heures, puis à nouveau filtrée pour séparer le gel des eaux de lavage.

Le gel d'hydroxyde d'étain ainsi obtenu a une teneur en eau variable suivant l'efficacité de la filtration mesurée par une perte au feu à 1000°C comprise entre 55 et 70 %.

### Préparation du précurseur de phase active

La teneur en eau du gel d'hydroxyde mesurée par perte au feu à 1000°C est de 57 % dans cet exemple.

On prépare par mélange du gel d'hydroxyde d'étain et du sel hydraté de chorure de Ru (III), 4414 g de précurseur contenant 1361 g de Sn0₂ anhydre et 397 g de Ru (sous forme de 957 g de RuCl₃, xH₂O à 43 % Ru).

La description suivante de la préparation du précurseur est ramenée à 1000 g de gel d'hydroxyde d'étain à 57 % d'eau.

A 1000 g de gel d'hydroxyde à 57 % d'eau soit 6,66 moles de SnO₂, on ajoute 2,9 moles de sel de RuCl₃, xH₂O à 43 % en Ru (soit 293,1 g de Ru).

Pour avoir un mélange rapide de rhydroxyde et du sel (environ 1 heure), on ajoute 260 g d'eau au mélange, ce qui ramène le gel d'hydroxyde à une teneur en eau de 65 % ;la pâte ainsi obtenue est très liquide.

On ajoute donc 79 g de silice Degussa-OX-50 comme épaississant, afin d'obtenir la viscosité adéquate pour l'enrobage.

On prépare ainsi 2548 g de précurseur de phase active.

Une variante consiste à mélanger le gel d'hydroxyde d'étain et le sel de Ru par agitation mécanique de longue durée (24h) sans ajout supplémentaire deau, ni de silice.

### Enrobage du précurseur de phase active sur le support Bille T375

Les 9 litres de billes T375 (11,25 kg) sont placées dans un drageoir, dont le volume, l'angle de l'axe de rotation et la vitesse de rotation sont choisis de façon à éviter à perte de bille par expulsion du drageoir et à obtenir un oeil dans le lit en rotation.

Le précurseur de phase active préparé précédemment est alors versé progressivement dans le drageoir en rotation.

L'enrobage des billes par le précurseur de phase active est description homogène.

On stoppe rajout de précurseur à l'apparition d'un aspect brillant, description humide sur les billes.

On verse alors dans le lit en rotation de la silice OX-50 Degussa.

Puis on maintient la rotation pendant environ 30 minutes afin de parfaire renrobage de la couche du précurseur par la silice.

Le catalyseur ainsi obtenu est mis à sécher en étuve sous vide de 540 mm Hg à 80°C. La hauteur du lit catalytique dans la nacelle est limitée à 2 cm.

Le séchage dure entre 5 heures et 16 heures.

Après séchage,le catalyseur est remis dans le drageoir pour pratiquer le deuxième enrobage de précurseur.

Le pourcentage souhaité en Ru est atteint après 6 enrobages de précurseur de phase active.

L'enchaînement des enrobages de précurseur de phase active et de silice pour la préparation du catalyseur I est résumé dans le tableau en annexe I.

Le catalyseur est prêt pour chargement dans le réacteur catatytique, où il sera activé par réduction sous hydrogène.

### CATALYSEUR II :

### Exemple 2 : Fabrication d'un lot de 9 litres de catalyseur 3.1 % Ru/Bille ex-argiles avec un rapport Sn/Ru de 3

Nous reproduisons la préparation précédente avec un rapport Sn/Ru = 3. La préparation du gel d'hydroxyde d'étain suit la même procédure.

Le gel d'hydroxyde d'étain présente dans ce cas après filtration une teneur en eau mesurée par sa perte au feu à 1000°C de 65 %.

Le mélange de ce gel d'hydroxyde avec le sel de Ru à un rapport Sn/Ru de 3 donne une pâte trop liquide pour être enrobée sur les billes.

Le séchage sous vide à 120°C de rhydroxyde d'étain permet d'abaisser la teneur résiduelle en eau à 57 % (perte au feu à 1000°C).

Le mélange de ce gel d'hydroxyde d'étain à 57 % d'eau avec le sel de chlorure de RU(III) suivant la procédure décrite précédemment (agitation pendant 24 heures) permet d'obtenir un précurseur de phase active à rapport Sn/Ru de 3 adéquat pour procéder à renrobage.

Une fluidité légèrement plus forte du précurseur par rapport à la préparation précédente, ainsi que l'usage d'une plus faible quantité de silice pour enrober et stabiliser les couches de précurseurs successives (149 g au lieu de 414 g) rendent nécessaire l'accumulation de 8 enrobages pour atteindre le pourcentage souhaité en Ru.

L'ensemble des opérations d'enrobage pour la préparation du catalys'eur Il est résumé dans le tableau en annexe II.

Les billes d'aluminium ont été traitées en 2 lots, comme décrit précédemment

### Exemple 3 : Exemple de préparation de fluoral hydraté CF₃CH(OH)₂ par réduction sélective de l'acide trifluoroacétipue CF₃COH TFA par l'hydrogène gaz en présence de catalyseur dont la phase active Ru₃SN₇ est déposée sur billes d'alumine.

Dans un réacteur en acier inoxydable (316L), de 30 cm de longueur, 28 g de catalyseur sont chargés. Après activation du catalyseur à température élevée sous courant d'hydrogène, le lit catalytique est alimenté toujours sous courant d'hydrogène par de l'acide trifluoroacétique à une température comprise entre 250 et 400°C, le rapport molaire H₂/TFA compris entre 1,5 et 4.

Le brut réactionnel est piégé dans un pot refroidi puis, après traitement usuel, est analysé par chromatographie en phase gazeuse:
- sélectivité en fluoral hydraté : 73 %
- conversion du TFA : 75%

Des essais similaires, en utilisant des phases support de différents types d'alumines. donnent des résultats analogues.

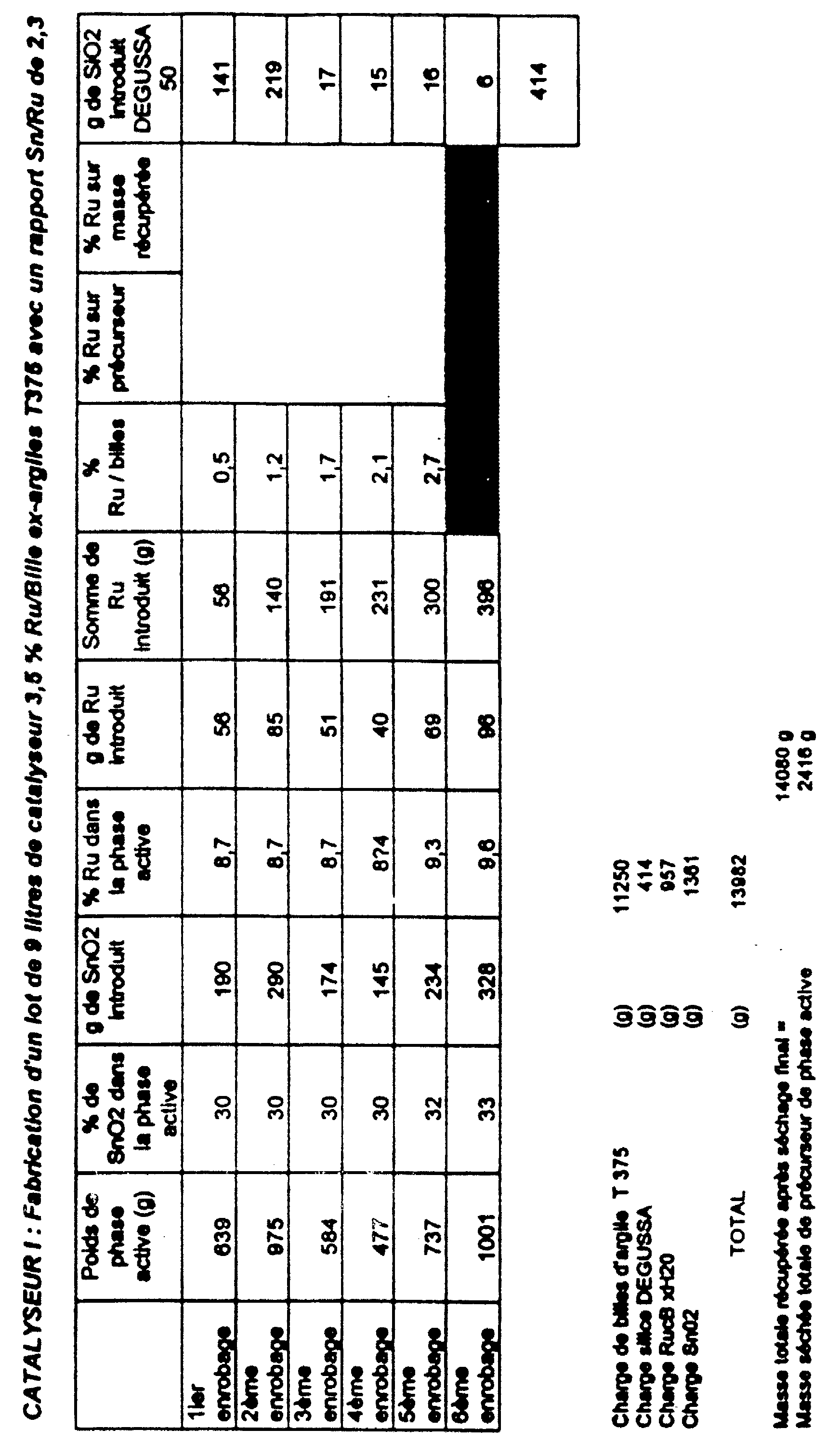

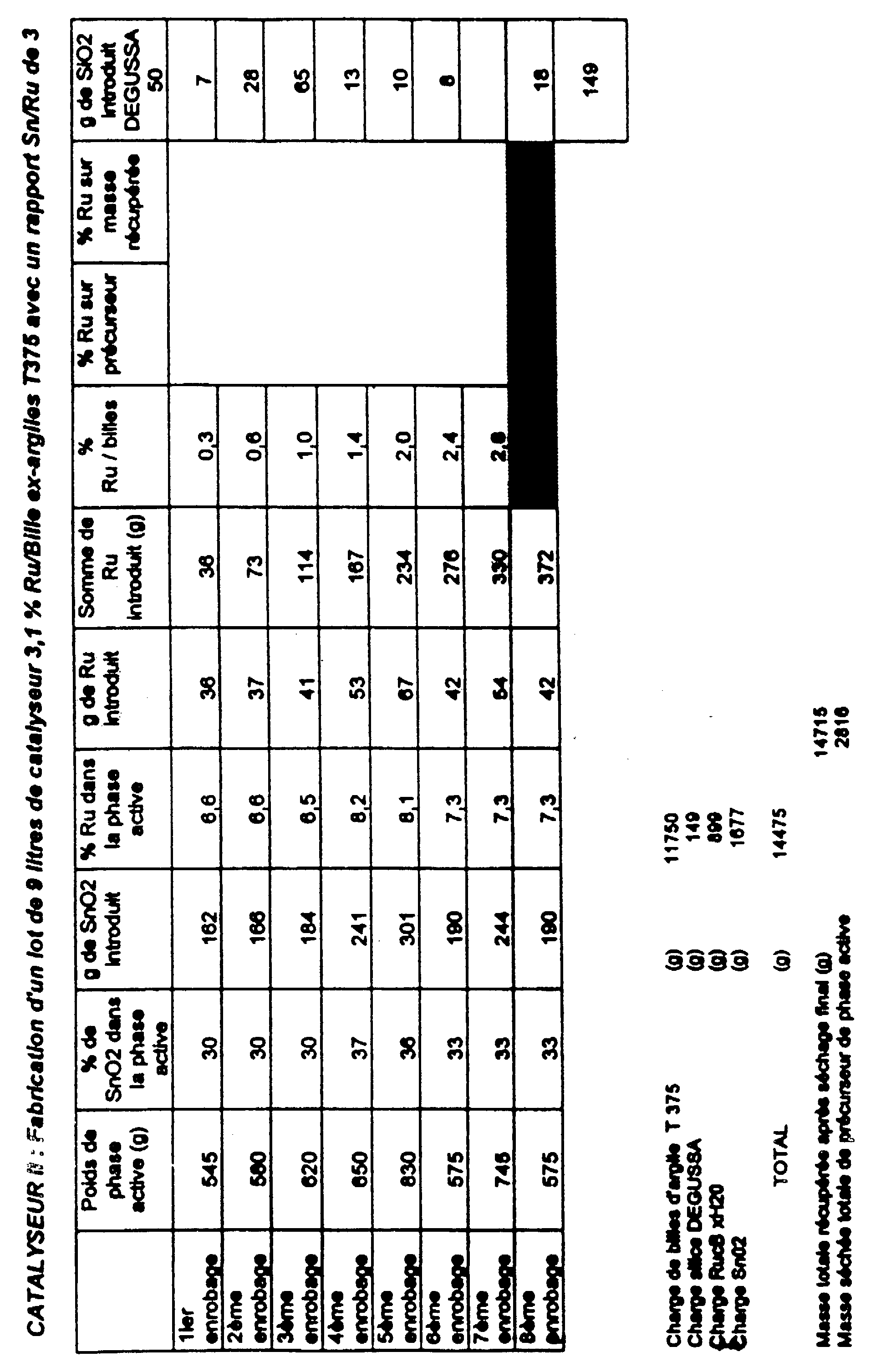

## Revendications

1. Composition utile comme catalyseur de réduction caractérisé par le fait qu'elle comporte un support qui comporte comme matériau constitutif au moins un oxyde choisi parmi les oxydes inertes ou susceptibles d'être rendu inertes vis à vis du mélange réactionnel et une phase métallique recouvrant au moins en partie ledit support comportant au moins en partie un intermétallique ruthénium-étain au moins partiellement sous forme du composé défini de Ru₃Sn₇, le rapport atomique Sn/Ru de la dite phase métallique étant au moins égal à 3/2 et au plus égal à 3, et par le fait que ladite phase contient au moins 50 % de phase Ru₃Sn₇.

2. Composition selon la revendication 1, caractérisé par le fait que le rapport Sn/Ru de la dite phase métallique est au plus égal à 5/2.

3. Composition selon les revendications 1 et 2, caractérisé par le fait que le rapport Sn/Ru de la dite phase métallique est au moins égal à 7/3.

4. Composition selon les revendications 1 à 3, caractérisé par le fait que ledit support présente une granulométrie telle que son d₂₀ soit au moins égal à 1 micromètres, avantageusement à 10 micromètres, de préférence à 50 micromètres.

5. Composition selon les revendications 1 à 4, caractérisé par le fait que ledit support présente une surface spécifique telle qu'elle soit au plus égale à 10 fois de préférence à 5 fois la surface dite géométrique.

6. Composition selon les revendications 1 à 5, caractérisé par le fait que lorsque la surface spécifique est au moins égale à deux fois la surface dite géométrique, à savoir celle calculée géométriquement en faisant l'hypothèse que toutes les particules sont sphériques, au moins 20%, avantageusement au moins 30 %, de préférence au moins 50%, de l'excès de surface est attribuable à des pores de diamètre moyen au moins égal à 0,5 micromètre, avantageusement au moins égal à 1 micromètre, de préférence au moins égal à 5 micromètres.

7. Composition selon les revendications 1 à 6, caractérisé par le fait que ledit support est un support susceptible d'être rendu inerte par l'action de l'acide fluorhydrique gazeux sans perdre sa géométrie externe.

8. Composition selon les revendications 1 à 7, caractérisé par le fait que ladite phase contient au moins 80%, de préférence au moins 90% de phase Ru3Sn7.

9. Composition selon les revendications 1 à 8, caractérisé par le fait que le rapport massique entre le support et la phase superficielle est comprise entre 0,5 % et 10 %, avantageusement de 0, 5 à 5%.

10. Composition selon les revendications 1 à 9, caractérisé par le fait que ledit support présente une teneur en silicium qui exprimée en SiO₂ soit au plus égale à 2/3 du poids total avantageusement au plus égale 1/4.

11. Composition selon les revendications 1 à 10, caractérisé par le fait qu'au moins 90 %, avantageusement au moins 95 %, de préférence 98 % du ruthénium présent sur le support est sous forme de ladite phase recouvrant ledit support.

12. Procédé de traitement des composés carboxyliques pour donner des aldéhydes ou leurs dérivés, caractérisé par le fait que l'on soumet ledit composé carboxylique à une hydrogénation en présence d'un catalyseur formé d'un support qui comporte comme matériau constitutif au moins un oxyde choisi parmi les oxydes inertes ou susceptibles d'être rendu inertes vis à vis du mélange réactionnel et une phase recouvrant au moins en partie ledit support comportant au moins en partie un intermétallique ruthénium-étain de composition Ru₃Sn₇, le rapport atomique Sn/Ru de la dite phase métallique étant au moins égal à 3/2 et au plus égal à 3, et pour le fait que ladite phase contient au moins 50% de phase Ru₃Sn₇

13. Utilisation (comme catalyseur de réduction) de composition(s) comportant un support qui comporte comme matériau constitutif au moins un oxyde choisi parmi les oxydes inertes ou susceptibles d'être rendu inertes vis à vis du mélange réactionnel et une phase recouvrant au moins en partie ledit support comportant au moins en partie un intermétallique ruthénium-étain de composition Ru₃Sn₇, le rapport atomique Sn/Ru de la dite phase métallique étant au moins égal à 3/2 et au plus égal à 3 et parle fait que ladite phase contient au moins 50 % de phase Ru₃Sn₇.

14. Procédé de production de catalyseur qui comporte un support et une phase métallique recouvrant au moins en partie ledit support, le rapport atomique Sn/Ru de la dite phase métallique étant au moins égal à 3/2 et au plus égal à 3, ladite phase contient au moins 50 % de phase Ru₃Sn₇ caractérisé par le fait qu'il comporte les étapes suivantes :
a) enrobage du support au moyen d'une solution ou d'une suspension d'espèces stannifère(s) et ruthénifère(s), le rapport atomique Sn/Ru étant au moins égal à 2,3 avantageusement à 3, de préférence à 4 ;
b) traitement à une température au moins égale à 400°, avantageusement à 450°C, sous une pression partielle d'hydrogène au moins égale à 1/2 10⁵ Pa, avantageusement comprise entre 1 et 10⁶ Pa, de préférence entre 1 et 2.10⁵ Pa caractérisé par le fait que le dit traitement est mené ce pendant une durée d'au moins environ cinq heures, de préférence au moins 10 heures.

## Patentansprüche

1. Als Reduktionskatalysator geeignete Zusammensetzung, dadurch gekennzeichnet, daß sie einen Träger, der als wesentliches Material mindestens ein Oxid, ausgewählt unter den gegenüber dem Reaktionsgemisch inerten oder inertisierbaren Oxiden und eine metallische Phase enthält, die diesen Träger mindestens teilweise bedeckt und die mindestens teilweise eine intermetallische Ruthenium-Zinn-Verbindung umfasst, die wenigstens teilweise in Form der definierten Zusammensetzung Ru₃Sn₇ vorliegt, wobei das Atomverhältnis Sn/Ru der metallischen Phase mindestens 3/2 und höchstens 3 ist und daß diese Phase mindestens 50 % Phase Ru₃Sn₇ enthält.

2. Zusammensetzung nach Anspruch 1, dadurch gekennzeichnet, daß das Verhältnis Sn/Ru der metallischen Phase höchstens 5/2 ist.

3. Zusammensetzung nach den Ansprüchen 1 und 2, dadurch gekennzeichnet, daß das Verhältnis Sn/Ru der metallischen Phase höchstens 7/3 ist.

4. Zusammensetzung nach den Ansprüchen 1 bis 3, dadurch gekennzeichnet, daß eine solche Korngröße aufweist, daß sein d₂₀ mindestens 1 Mikrometer, vorteilhaft 10 Mikrometer und vorzugsweise 50 Mikrometer ist.

5. Zusammensetzung nach den Ansprüchen 1 bis 4, dadurch gekennzeichnet, daß der Träger eine spezifische Oberfläche aufweist, die höchstens das 10-fache, vorzugsweise höchstens das 5-fache der sogenannten geometrischen Oberfläche ist.

6. Zusammensetzung nach den Ansprüchen 1 bis 5, dadurch gekennzeichnet, daß, wenn die spezifische Oberfläche mindestens gleich dem Zweifachen der sogenannten geometrischen, d.h. der unter der theoretischen Voraussetzung, dass alle Partikel sphärisch sind, geometrisch berechneten Oberfläche ist, mindestens 20 %, vorteilhaft mindestens 30 % und vorzugsweise mindestens 50 % des Oberflächenüberschusses Poren eines mittleren Durchmessers von mindestens 0,5 Mikrometer, vorteilhaft mindestens 1 Mikrometer und vorzugsweise mindestens 5 Mikrometer zugeordnet werden kann.

7. Zusammensetzung nach den Ansprüchen 1 bis 6, dadurch gekennzeichnet, daß der Träger ein Träger ist, der durch Einwirkung von gasförmiger Fluorwasserstoffsäure inert gemacht werden kann, ohne seine äußere Geometrie zu verlieren.

8. Zusammensetzung nach den Ansprüchen 1 bis 7, dadurch gekennzeichnet, daß die besagte Phase mindestens 80 %, vorzugsweise mindestens 90 % Phase Ru₃Sn₇ enthält.

9. Zusammensetzung nach den Ansprüchen 1 bis 8, dadurch gekennzeichnet, daß das Massenverhältnis des Trägers zur Oberflächenphase zwischen 0,5 % und 10 %, vorteilhaft 0,5 bis 5 % beträgt.

10. Zusammensetzung nach den Ansprüchen 1 bis 9, dadurch gekennzeichnet, daß der Träger einen Siliciumgehalt aufweist, der, ausgedrückt als SiO₂, höchstens 2/3, vorteilhaft höchstens 1/4 des Gesamtgewichts ist.

11. Zusammensetzung nach den Ansprüchen 1 bis 10, dadurch gekennzeichnet, daß mindestens 90 %, vorteilhaft mindestens 95 %, vorzugsweise 98 % des auf dem Träger befindlichen Rutheniums in Form der Phase vorliegt, die den Träger bedeckt.

12. Verfahren zur Behandlung von Carboxylverbindungen zur Bildung von Aldehyden oder deren Derivaten, dadurch gekennzeichnet, daß man die Carboxylverbindung einer Hydrierung in Gegenwart eines Katalysators unterwirft, der aus einem Träger gebildet ist, der als wesentlichen Bestandteil mindestens ein Oxid, ausgewählt unter den gegenüber dem Reaktionsgemisch inerten oder inertisierbaren Oxiden und mindestens eine metallische Phase umfasst, die diesen Träger mindestens teilweise bedeckt und die mindestens teilweise eine intermetallische Ruthenium-Zinn-Verbindung der Zusammensetzung Ru₃Sn₇ enthält, wobei das Atomverhältnis Sn/Ru der metallischen Phase mindestens 3/2 und höchstens 3 ist und diese Phase mindestens 50 % Phase Ru₃Sn₇ enthält.

13. Verwendung der Zusammensetzung(en), die einen Träger, der als wesentliches Material mindestens ein Oxid, ausgewählt unter den gegenüber dem Reaktionsgemisch inerten oder inertisierbaren Oxiden und eine metallische Phase umfasst/umfassen, die diesen Träger mindestens teilweise bedeckt und mindestens teilweise eine intermetallische Ruthenium-Zinn-Verbindung umfasst, die mindestens teilweise in Form der definierten Zusammensetzung Ru₃Sn₇ vorliegt, wobei das Atomverhältnis Sn/Ru der metallischen Phase mindestens 3/2 und höchstens 3 ist, wobei diese Phase mindestens 50 % Ru₃Sn₇ -Phase enthält (als Reduktionskatalysator).

14. Verfahren zur Herstellung eines Katalysators, der einen Träger und eine metallische Phase umfasst, die diesen Träger mindestens teilweise bedeckt, wobei das Atomverhältnis Sn/Ru dieser metallischen Phase mindestens 3/2 und höchstens 3 ist und diese Phase mindestens 50 % Ru₃Sn₇ -Phase enthält, dadurch gekennzeichnet, daß es die folgenden Schritte umfasst:
a) Umhüllen des Trägers mit einer Lösung oder Suspension Zinn und Ruthenium enthaltender Verbindung(en), wobei das Atomverhältnis Sn/Ru mindestens gleich 2,3, vorteilhaft 3 und vorzugsweise 4 ist;
b) Behandlung bei einer Temperatur von mindestens 400 °, vorteilhaft 450 °C bei einem Wasserstoff-Partialdruck von mindestens gleich 1/2·10⁵ Pa, vorteilhaft zwischen 1 und 10⁶ Pa, vorzugsweise zwischen 1 et 2·10⁵ Pa, dadurch gekennzeichnet, daß die Behandlung über eine Dauer von mindestens ungefähr 5 Stunden, vorzugsweise mindestens 10 Stunden fortgeführt wird.

## Claims

1. Composition which can be used as reduction catalyst, characterized in that it comprises a support whose constituent material comprises at least one oxide, selected from oxides which are inert or can be made inert relative to the reaction mixture, and a metallic phase at least partially covering the said support, of which at least part comprises an intermetallic ruthenium-tin compound at least some of which is in the form of the defined compound Ru₃Sn₇, the Sn/Ru atomic ratio of the said metallic phase being at least 3/2 and at most 3, and in that the said phase contains at lease 50 % of Ru₃Sn₇ phase.

2. Composition according to claim 1, characterized in that the Sn/Ru ratio of the said metallic phase is at most 5/2.

3. Composition according to claims 1 and 2, characterized in that the Sn/Ru ratio of the said metallic phase is at least 7/3.

4. Composition according to claims 1 to 3, characterized in that the said support has a particle size such that its d₂₀ is at least 1 micrometre, advantageously 10 micrometres, preferably 50 micrometres.

5. Composition according to claims 1 to 4, characterized in that the said support has a specific surface area such that it is at most 10 times, preferably 5 times, the geometric surface area.

6. Composition according to claims 1 to 5, characterized in that, when the specific surface area is at least twice the geometric surface area, namely that calculated geometrically on the hypothesis that all of the particles are spherical, at least 20 %, advantageously at least 30 %, preferably at least 50 %, of the surface excess can be attributed to pores with a mean diameter of at least 0.5 micrometre, advantageously of at least 1 micrometre, preferably of at least 5 micrometres.

7. Composition according to claims 1 to 6, characterized in that the said support is a support which can be made inert by the action of gaseous hydrofluoric acid without losing its external geometry.

8. Composition according to claims 1 to 7, characterized in that the said phase contains at least 80 %, preferably at least 90 %, of Ru₃Sn₇ phase.

9. Composition according to claims 1 to 8, characterized in that the mass ratio between the support and the surface phase is between 0.5 % and 10 %, advantageously from 0.5 to 5 %.

10. Composition according to claims 1 to 9, characterized in that the said support has a silicon content which, expressed as SiO₂, is at most 2/3 of the total weight, advantageously at most 1/4.

11. Composition according to claims 1 to 10, characterized in that at least 90 %, advantageously at least 95 %, preferably 98 %, of the ruthenium present on the support is in the form of the said phase covering the said support.

12. Process for the treatment of carboxylic compounds to give aldehydes or derivatives thereof, characterized in that the said carboxylic compound is subjected to hydrogenation in the presence of a catalyst formed from a support whose constituent material comprises at least one oxide, selected from oxides which are inert or can be made inert relative to the reaction mixture, and a phase at least partially covering the said support, of which at least part comprises an intermetallic ruthenium-tin compound of composition Ru₃Sn₇, the Sn/Ru atomic ratio of the said metallic phase being at least 3/2 and at most 3, and in that the said phase contains at least 50 % of Ru₃Sn₇ phase.

13. Use (as reduction catalyst) of composition(s) comprising a support whose constituent material comprises at least one oxide, selected from oxides which are inert or can be made inert relative to the reaction mixture, and a phase at least partially covering the said support, of which at least part comprises an intermetallic ruthenium-tin compound of composition Ru₃Sn₇, the Sn/Ru atomic ratio of the said metallic phase being at least 3/2 and at most 3, and in that the said phase contains at least 50 % of Ru₃Sn₇ phase.

14. Process for producing a catalyst comprising a support and a metallic phase at least partially covering the said support, the Sn/Ru atomic ratio of the said metallic phase being at least 3/2 and at most 3, the said phase contains at least 50 % of Ru₃Sn₇ phase, characterized in that it comprises the following steps:
a) coating of the support by means of a solution or a suspension of stanniferous and rutheniferous species, the Sn/Ru atomic ratio being at least 2.3, advantageously 3, preferably 4;
b) treatment at a temperature of at least 400°, advantageously 450°C, under a hydrogen partial pressure of at least 1/2 × 10⁵ Pa, advantageously between 1 and 10⁶ Pa, preferably between 1 and 2 × 10⁵ Pa, characterized in that the said treatment is conducted for a period of at least approximately five hours, preferably at least 10 hours.
